# EUROPÄISCHE PATENTSCHRIFT

(11) **EP 0 982 348 B1**
(45) Veröffentlichungstag und Bekanntmachung des Hinweises auf die Patenterteilung: **26.11.2003**
(21) Anmeldenummer: 99116059.9
(22) Anmeldetag: 16.08.1999
(51) Int. Cl.: C08G 77/14, A61K 47/34

(54) **Polymerzusammensetzung und Verfahren zu deren Herstellung**
Polymer composition and process for its manufacture
Composition polymère et procédé d'obtention

(30) Priorität: 17.08.1998 DE 19837234
(43) Veröffentlichungstag der Anmeldung: 01.03.2000
(73) Patentinhaber: Freie Universität Berlin, 14195 Berlin (DE)
(72) Erfinder: Roth, Christoph Dr., 06118 Halle (DE); Pietras, Ulrike Dr., 14163 Berlin (DE); Woitschig, Gerhard, 06766 Wolfen (DE)
(74) Vertreter: Hertz, Oliver, Dr.

(56) Entgegenhaltungen:
- EP-A- 0 399 827
- EP-A- 0 699 431
- EP-A- 0 718 340
- US-A- 4 678 468
- US-A- 5 071 978
- SCHULDT U ET AL: "VERGLEICHENDE UNTERSUCHUNGEN ZUR SILYLIERUNG VON CELLULOSE MIT MONO- UND MULTI-FUNKTIONELLEN ORGANOSILANEN" PAPIER, DAS,DE,EDUARD ROETHER KG. DARMSTADT, Bd. 48, Nr. 1, Seite 3-05,8-10, XP000418284 ISSN: 0031-1340

## Beschreibung

Die Erfindung betrifft eine Polymerzusammensetzung aus biokompatiblen und/oder bioabbaubaren Polymeren, die durch Siliziumoxid-Bausteine vernetzt sind und als Trägermaterial, z. B. als Matrix oder Umhüllung für biologisch aktive Agenzien, vorzugsweise pharmazeutische Wirkstoffe dient, deren Freigabe kontrolliert werden soll. Die Erfindung betrifft ebenfalls ein Verfahren zur Herstellung der Polymerzusammensetzung sowie der Polymer-Wirkstoff-Formulierung und gibt Verwendungszwecke hierfür an.

Im medizinischen Bereich wird für eine sichere, patientenorientierte Therapie angestrebt, die Darreichungsformen von Arzneimitteln gezielt zu verändern. Die orale Arzneistoffapplikation ist z.B. durch den Einfluß der Nahrung auf die Resorption, intra- und interindividuelle Schwankungen der Plasmaspiegel, Beeinträchtigung der Bioverfügbarkeit durch den "First-Pass-Effekt" und den Abbau von Wirkstoffen im Gastrointestinaltrakt nachteilig. Zur Kompensation dieser Nachteile wird häufig die Wirkstoffdosis überhöht, was jedoch unerwünschte lokale oder systemische Wirkungen zur Folge haben kann. Bei parenteraler Applikation erfordern kurze Halbwertszeiten eine mehrfach tägliche Injektion, was für den Patienten eine erhebliche, ungern tolerierte Beeinträchtigung darstellt. Zusätzlich stehen durch die Ergebnisse der biotechnologischen Forschung zahlreiche neuartige Wirkstoffe, wie Oligonukleotide, Peptide und Proteine zur Verfügung, die aufgrund der schnellen Hydrolyse im Magen-Darmtrakt, bzw. aufgrund ihrer kurzen Halbwertszeit auf konventionellem Weg nicht oder nur eingeschränkt verabreichbar sind, da therapeutisch wirksame Blutspiegel über einen längeren Zeitraum nicht erreicht werden. Um konstante, wirksame Blutspiegel zu erreichen, ist die Entwicklung von parenteralen Depotsystemen sinnvoll, bei denen der Wirkstoff an einen polymeren Träger gebunden wird. Dieser schützt den Wirkstoff vor hydrolytischen und enzymatischen Einflüssen des umgebenden Mediums und garantiert über einen gewissen Zeitraum ein gewünschtes Freigabeprofil. Durch Variation, d. h. hydrophile oder lipophile Modifikation der Trägeroberflächen ist es teilweise möglich, eine zielgerichtete Medikation im Organismus zu erreichen, so daß einerseits die therapeutische Dosierung am Zielorgan optimiert und die systemische Belastung mit unnötig hohen Wirkstoffdosen reduziert wird.

In der Fach- und Patentliteratur werden eine Vielzahl von parenteralen Depotsystemen in Form von Mikro-/Nanokapseln oder Mikro-/Nanopartikeln oder Mini-Implantaten beschrieben, die den Wirkstoff kontinuierlich freisetzen (siehe z. B. "Microspheres and Drug Therapy, Pharmaceutical, Immunological and Medical Aspects" ed. S. S. Davies, L. Illum, J. G. McVie, E. Tomlinson Elsevier, Amsterdam 1984; "Protein Delivery", ed. L. M. Sanders, R. W. Hendren; Plenum Press, NY, London, 1997). Als Polymere werden i. d. R. Milchsäure, Glykolsäure und deren Copolymere verwendet. Trotz intensiver Forschung auf diesem Gebiet, sind jedoch bisher nur wenige parenterale Depotformen auf dem Markt.
Dies liegt daran, daß die bisherigen Systeme noch zahlreiche Nachteile haben wie bspw.:
ein unbefriedigendes Freisetzungsprofil bedingt durch hydrophobe Polymereigenschaften, die eine geringe Quellung des Polymeren und damit nur eine eingeschränkte Diffusion des Wirkstoffs nach außen zulassen, Verschiebungen des pH-Wertes innerhalb der Matrix durch hydrolytischen Abbau, was zur Denaturierung des Wirkstoffes und zu einem beschleunigten Polymerabbau auch innerhalb der Matrix mit ungesteuerter Wirkstofffreigabe führt, Auftreten eines "initial burst", d. h. einer hohen Wirkstoffabgabe unmittelbar nach Applikation durch an der Oberfläche der Darreichungsform haftenden Wirkstoff,
eine eingeschränkte Biokompatibilität durch Verwendung toxikologisch bedenklicher bzw. großer Mengen chemischer Lösungs- und Härtungsmittel bei der Herstellung, die nicht restlos aus dem Endprodukt entfernt werden können, unerwünschte Reaktionen hervorrufen und die Sicherheit und Unbedenklichkeit beeinträchtigen.
Der Einsatz von Biopolymeren als Matrixmaterialien für Wirkstoffapplikationen ist bisher teilweise nur eingeschränkt realisierbar, da die Rohstoffchargen in Bezug auf Reinheit und Zusammensetzung stark variieren. Dies führt zu nicht reproduzierbaren Ergebnissen und Qualitätseinbußen, die insbesondere bei pharmazeutischen Anwendungen, d. h. in der Medizin und Tiermedizin inakzeptabel sind.

Neben der Anwendung von bioabbaubaren Polymeren in Wirkstoffträgersystemen befinden sich in diesem Anwendungsbereich auch rein anorganische Materialien auf Sol-Gel Basis verstärkt in der Entwicklung. Sie dienen bspw. zur Verkapselung oder Immobilisierung von Enzymen oder Antikörpern, als Carrier pharmazeutischer Wirkstoffe bzw. für den DNA-Transfer oder als Träger von Zellen, bspw. Inselzellen, die intraperitoneal implantiert über Monate Insulin synthetisieren und kontinuierlich freisetzen (siehe z. B. "Affinity of Antifluorescein Antibodies Encapsulated Within a Transparent Sol-Gel Glass"; R. Wang et al.; Anal.Chem. 1993, 65:2671-75; "Silica particles as pharmaceutical carrier systems"; M. Sameti et al., bzw. C. Kneuer et al. in Progress Report 97/98 Dep. Biopharm. and Pharm.Tech., Universität des Saarlandes; "Silica Sol-Gel Encapsulation of Pancreatic Islets" K. P. Peterson et al.;Proc. Soc. Exp. Biol. Med. 1998, Sep;Vol 218(4):365-9)).

Der Vorteil der Verwendung rein anorganischer Silica-Matrizen besteht darin, daß diese inert, toxikologisch unbedenklich, einfach und umweltfreundlich herstellbar sind und hinsichtlich ihrer Reinheit, reproduzierbaren Eigenschaften und der Vielzahl an zur Verfügung stehenden Materialien, die kombiniert werden können, zahlreiche Möglichkeiten für neue Applikationsformen eröffnen. Der Nachteil besteht darin, daß die anorganischen Bestandteile nicht bioabbaubar sind und wie im Fall der verkapselten Inselzellen entweder chirurgisch entfernt werden müssen oder möglicherweise im Organismus akkumuliert werden, bzw. zu Problemen bei der Ausscheidung führen.

Aus DE-OS 44 16 003 und EP 0 680 753 ist ein Verfahren bekannt, bei dem organische Flüssigkeiten oder Öle durch ein Sol-Gel-Verfahren (s. "Sol-Gel-Science", C.J. Brinker, G.W. Scherer, Academic Press Inc., San Diego 1990) in Metalloxide inkorporiert werden. Zur Herstellung von Pulvern oder Filmen wird die zu inkorporierende Flüssigkeit in einem organisch - wäßrigen Metalloxid-Sol gelöst, das durch saure oder alkalische Hydrolyse von Metallalkoxiden (Silizium-, Aluminium- oder Titanalkoxiden oder deren Gemische) in einem mit Wasser mischbaren organischen Lösungsmittel erhalten wird. Die derart beladenen Metalloxid-Sole werden durch Trocknung in den Gel-Zustand überführt und als rieselfähiges Pulver bzw. als Schicht auf einem Schichtträger verwendet. Nach diesem Verfahren hergestellte Arzneizubereitungen oder Kosmetika gestatten eine verzögerte Freisetzung der inkorporierten Flüssigkeit in das umgebende Medium. Die Erfindung beschränkt sich auf die Anwendung in transdermalen Systemen (siehe auch: "Sol-Gel Carrier Systems for Controlled Drug Delivery", H. Böttcher et al.; J. Sol-Gel Science and Technology 13, 277-81 (1998)).

Von Seiten der pharmazeutischen Forschung besteht somit großes Interesse an neuen Polymeren, mit denen die bisherigen Nachteile beseitigt werden, möglicherweise Individualerfordernisse berücksichtigt werden können oder auf deren Grundlage völlig neue therapeutische Darreichungsformen entstehen. In der Fach- und Patentliteratur finden sich zahlreiche Publikationen zu Formulierungen auf der Basis von rein organischen bzw. rein anorganischen Polymeren. Bisher finden sich keine Hinweise auf Kombinationen der beiden Komponenten in den genannten Anwendungsbereichen, wie sie Gegenstand des vorliegenden Patentes sind. Als Hybridmaterialien für biomedizinische Anwendungen sind lediglich Kombinationen des Biopolymers Chitosan mit einem nicht-funktionellen Silan (Tetraethoxysilan) bekannt ( siehe: "Preparation and Blood Compatibility of new Silica-Chitosan Hybrid Biomaterials", H. Chen et al.;Artif Cells Blood Substit Immobil Biotechnol 1998 Jul; 26(4): 431-6; "pH Sensitive Membrane Using TEOS-Chitosan Hybrid IPN"; H. Y. Park et al., Proc. Int'1. Symp. Control. Rel. Bioact. Mater., 25 (1998)).

Polymerzusammensetzungen mit eingebauten Siliziumatomen sind zur Herstellung von kosmetischen Basismaterialien, Implantatmaterialien oder technischen Hilfsstoffen in EP-A-0 699 431, US-A-5 071 078, EP-A-0 3 999 827, EP-A-0 718 340 und US-A-4 678 468 beschrieben.

Aufgabe der Erfindung ist es somit, eine Polymerzusammensetzung anzugeben, mit der die genannten Nachteile herkömmlicher Systeme überwunden werden, die einen erweiterten Anwendungsbereich besitzt, wobei insbesondere eine Polymerzusammensetzung als Trägermaterial für Wirkstoffe geschaffen werden soll, dessen Eigenschaften als Wand- oder Matrixmaterial variiert, definiert, evaluiert und dem Anwendungszweck angepaßt werden können. Die Aufgabe der Erfindung ist es auch, ein Verfahren zur Herstellung einer derartigen Polymerzusammensetzung und Verwendungen dafür anzugeben.

Die Aufgabe wird insbesondere gelöst durch eine Polymerzusammensetzung, die wasserlösliche Polymere enthält, die durch kovalent gebundene Siliziumoxid-Bausteine vernetzt sind und die als bioabbaubares und biokompatibles Trägermaterial für biologisch aktive Agenzien, bevorzugt pharmazeutische Wirkstoffe dient. Gleichzeitig wird ein Verfahren zur Herstellung der Polymerzusammensetzung angegeben, bei dem eine wäßrige Polymerlösung mit funktionellen Silanen, bevorzugt funktionellen Alkoxysilanen, deren Hydrolysaten oder den aus ihnen gebildeten funktionellen Siliziumoxidsolen oder Mischsolen aus funktionellen und nicht-funktionellen Solen versetzt wird.

Als wasserlösliche Polymere werden bevorzugt biologisch abbaubare, biokompatible Polymere, wie z.B. hydrokolloide Biopolymere verwendet.
Beispiele für wasserlösliche Polymere sind unter anderem:
Polysaccharide (z. B. Stärke, Dextrine, Lactosen ...)
Proteine (z. B. Gelatine Molmasse 20.000-400.000g/Mol, Albumin, Kollagen ...)
Zellulosederivate (z.B. Hydroxypropylzellulose, Zellulosesulfat, Carboxymethylzellulose, Hydroxyethylzellulose ...)

Zur Herstellung der erfindungsgemäßen Polymerzusammensetzungen wird eine Polymerlösung mit einem oder mehreren Alkoxysilanen, deren Hydrolysaten oder den aus ihnen gebildeten Siliziumoxidsolen versetzt. Die Polymerlösung ist eine verdünnte Lösung des wasserlöslichen Polymers. Der Polymeranteil in der Lösung beträgt rund 0,5 - 10 Gewichtsprozent.

Die Alkoxysilane besitzen die allgemeine Formel Si(OR)ₓR¹_{Y} . R kann gleichartig oder verschieden zusammengesetzt sein und bedeutet einen C₁-C₄-Alkylrest. R¹ kann ebenfalls gleichartig oder verschieden zusammengesetzt sein und stellt einen Alkyl-, Aryl- oder Alkylarylrest dar, der eine funktionelle Gruppe tragen kann. Eine funktionelle Gruppe ist beispielsweise eine Epoxygruppe, x steht für eine Zahl im Bereich von 1 - 4, y steht für eine Zahl im Bereich von 0 - 3, wobei ein Mindestgehalt an funktionellem Silan von 0,1 Mol vorliegen muß.

Aus einem oder mehreren verschiedenen Alkoxysilanen der allgemeinen Formel Si(OR)ₓR¹_{Y} oder deren Hydrolysaten wird ein funktionelles Siliziumdioxidsol gebildet, wobei mindestens eine Komponente (Alkoxysilane oder Hydrolysate) Träger einer funktionellen Gruppe sein muß. Der Polymerlösung können zusätzlich Mischsole aus funktionellen und nicht-funktionellen Siliziumoxidsolen zugesetzt werden, wobei nicht-funktionelle Sole aus Alkoxysilanen Si(OR)ₓR¹_{Y} oder deren Hydrolysaten dargestellt werden und R¹ kein Träger einer funktionellen Gruppe ist. Die nicht-funktionellen Siliziumoxidsole können durch ein oder verschiedene Alkoxysilane der genannten Formel oder deren Hydrolysate gebildet werden.

Der Anteil der Alkoxysilane ,bevorzugt epoxidfunktionelle Alkoxysilane, deren Hydrolysate oder der aus ihnen gebildeten, funktionellen Siliziumoxidsole in der Polymerzusammensetzung liegt im Bereich von 0,1 - 40 Gewichts-%, vorzugsweise 10 - 20 Gewichts-%. Der Anteil wird in Abhängigkeit vom eingesetzten wasserlöslichen Polymeren ausgewählt. Der Anteil der epoxidfunktionellen Gruppen in den Ausgangssolen liegt zwischen 0,05 - 35 Mol-% ,vorzugsweise im Bereich von 1 - 20 Mol-%, bezogen auf Si-Gruppen.

Zusätzlich zu den funktionellen, bevorzugt epoxidfunktionellen Solen können als anorganische Komponente auch oberflächenmodifizierte inerte Sole oder Dispersionen verwendet werden, deren Herstellung in bekannter Weise als Lösung oder Feststoff durch Beladung mit funktionellen, bevorzugt epoxidfunktionellen Silanen erfolgt.

Die Bildung der Polymerzusammensetzung erfolgt derart, daß in der Hauptkette und/oder in den Nebenketten der wasserlöslichen Polymeren vorhandene und/oder durch zusätzliche chemische Modifizierung erzeugte funktionelle Gruppen (wie z.B. -OH, -NH₂ oder -COOH ) mit den entsprechenden funktionellen Gruppen der Alkoxysilane, deren Hydrolysaten oder den aus ihnen gebildeten Siliziumoxidsolen reagieren. Zusätzlich finden Reaktionen zwischen den funktionellen Gruppen der Siliziumverbindungen statt. Hierzu zählen insbesondere Kondensationsreaktionen, die sowohl im sauren oder auch im basischen Milieu stattfinden können. Es können ferner intermolekulare Reaktionen der wasserlöslichen Polymeren stattfinden, so daß diese mit sich selbst Vernetzungspunkte bilden. Die einzelnen Vernetzungs- und gegebenenfalls Kettenwachstumsreaktionen finden in Abhängigkeit von den Reaktionsbedingungen zeitlich parallel oder versetzt statt.

Die Herstellung von funktionellen Solen und Solgemischen aus funktionellen und nichtfunktionellen Silanen erfolgt in bekannter Weise durch eine Hydrolyse von funktionellen Silanen oder Gemischen aus funktionellen und nichtfunktionellen Silanen und anschließender Kondensation in Wasser/ Ethanol- Gemischen (siehe "Sol-Gel-Science", C.J. Brinker, G.W. Scherer, Academic Press Inc., San Diego 1990)

Beispiele für Zusammensetzungen von Mischsolen sind:
1.) 50 Teile Tetraethoxysilan
   50 Teile Glycidoxypropyltriethoxysilan
2.) 75 Teile Glycidoxypropyltrimethoxysilan
   25 Teile Methyltriethoxysilan
3.) 25 Teile Glycidoxypropyltriethoxysilan
   50 Teile Tetraethoxysilan
   25 Teile Methyltriethoxysilan
4.) 50 Teile Glycidoxypropyltriethoxysilan
   25 Teile Dimethyldiethoxysilan
   25 Teile Tetraethoxysilan

Die Herstellung der erfindungsgemäßen Polymerzusammensetzung erfolgt in einer Lösung und kann je nach Anwendungszweck unterschiedlich aufgearbeitet werden. Eine Feststoffisolierung erfolgt durch einen Trocknungsvorgang, wie z.B. eine Wirbelschichttrocknung, eine Sprühtrocknung oder eine Gefriertrocknung.

Die erfindungsgemäß hergestellten Polymerzusammensetzungen werden vorzugsweise als Träger von Wirkstoffen ( biologisch aktiven Agenzien ) oder als Umhüllungsmaterial verwendet. Die Polymerzusammensetzung kann somit entweder Wandmaterial für einen an sich bekannten Mikroverkapselungsprozeß sein, bei dem der Wirkstoff oder das biologische Material verkapselt wird oder an deren Wandung der Wirkstoff oder das biologische Material gekoppelt ist oder Matrixmaterial für Partikel im Mikro-/Nanobereich oder für Mini-Implantate oder für Schichten sein. Die Einbringung oder Inkorporierung der Wirkstoffe erfolgt simultan zur Bildung der Polymerzusammensetzung oder nachträglich in die vorgefertigten Polymerzusammensetzungen.

Für eine Einlagerung der Wirkstoffe simultan zur Bildung der Polymerzusammensetzung werden die Wirkstoffe (z.B. biologisch aktive Agenzien) in Gegenwart des wasserlöslichen Polymeren, gelöst oder fein dispergiert. Anschließend wird die komplementäre Komponente , vorzugsweise die beschriebenen funktionellen Alkoxysilane, deren Hydrolysate oder die aus ihnen gebildeten funktionellen Siliziumoxidsole oder funktionelle/nichtfunktionelle Siliziumoxid-Mischsole, zugegeben.

Zur nachträglichen Einlagerung von Wirkstoffen wird beispielsweise wie folgt verfahren: Mit den vorgefertigten Polymerzusammensetzungen werden in an sich bekannter Weise biologisch aktive Substanzen nach herkömmlichen Mikroverkapselungsverfahren (wie z.B. Phasentrennung aus wäßrigen Systemen) umhüllt, oder sie werden als Wirkstoffträger verwendet, auf denen der Wirkstoff reversibel chemisch oder physikalisch gebunden ist.

Gegenstand der Erfindung ist nicht nur die beschriebene Verfahrensweise zur Einlagerung biologisch aktiver Agenzien in bioverträglichen Medien sowie deren anschließende gezielte Freisetzung z.B. im Organismus, sondern auch die Verwendung der Polymerzusammensetzung, gegebenenfalls ohne Wirkstoffbeladung als Werkstoff z.B. in Schichtform.

Die Anwendung der Erfindung ist nicht auf die Medizin oder Pharmazie beschränkt, sondern auch in der Kosmetik oder Lebensmitteltechnik einsetzbar, um beispielsweise bestimmte Wirkstoffe oder Substanzen gegenüber der Umgebung schützend abzudecken oder einzuschließen. Außer im biologischen Bereich liegen weitere Anwendungsmöglichkeiten in nichtbiologischen, z.B. technischen Bereichen, in denen eine erhöhte Stabilität (chemisch, mechanisch, thermisch) der betreffenden Polymere bzw. die Steuerung der Freigabe eines gebundenen Agens oder dessen Schutz vor dem umgebenden Medium gewünscht ist.

Vorteilhafterweise können die getrockneten Polymerzusammensetzungen problemlos gelagert und transportiert werden. Es zeigt sich insbesondere im Vergleich mit herkömmlichen Biopolymeren eine erheblich reduzierte Feuchteempfindlichkeit. Zur weiteren Verarbeitung werden die Feststoffe anwendungsabhängig vorgequollen, geschmolzen und anschließend wie die bei der Herstellung gebildeten Lösungen weiterverarbeitet.

Eine bevorzugte Anwendung der Erfindung liegt in der Schaffung von Trägersystemen für Wirkstoffe in der Medizin und Tiermedizin, insbesondere für Applikationen entsprechend den unten angegebenen Beispielen.

Durch die Kombination. der biokompatiblen, bioabbaubaren Polymere mit biologisch inerten, nicht abbaubaren Siliziumoxidmolekülen werden universell einsetzbare Polymerzusammensetzungen geschaffen, mit denen wesentliche Nachteile der reinen Biopolymere sowie der rein anorganischen Siliziumoxid-Polymere beseitigt werden.

Als besondere Vorteile sind zu nennen:
Die definierte Festlegung des Vernetzungsgrades ermöglicht die Unterdrückung des "initial burst", sowie eine reproduzierbare Variation eines gewünschten Freigabeprofils ( siehe Grafiken 1-2, 5)
Die Polymerzusammensetzung ist hydrophil und kann durch den Einbau der wasserlöslichen Biopolymere hydrolytisch abgebaut werden und den Wirkstoff kontinuierlich freigeben.
Der Abbau erfolgt durch sukzessive Quellung und Erosion
Der Abbau erfolgt ohne pH-Wert Verschiebung
Der anorganische Anteil der Polymerzusammensetzung ist inert und toxikologisch unbedenklich
Der Anteil an anorganischer Struktur in der Polymerzusammensetzung kann soweit minimiert werden, daß nach Hydrolyse der Biopolymere eine vollständige Ausscheidung aus dem Organismus möglich ist
Die mechanische, chemische und physikalische Stabilität der Biopolymeren wird erhöht
Die Freigabecharakteristik wird durch die Lagerung nicht verändert (siehe Grafiken 3-4)
Die Herstellung der Polymerzusammensetzung erfolgt mit Standardmethodik, umweltfreundlichen und milden Prozeßtechnologien. Der Einsatz von Chemikalien zur Lösung und Härtung entfällt, weshalb das Endprodukt frei von derartigen Substanzen ist.
Durch Variation der organischen und anorganischen Ausgangspolymere ist eine Vielzahl von Polymerzusammensetzungen möglich

Die aufgeführten Vorteile zeigen, daß die erfindungsgemäße Polymerzusammensetzung sich nicht nur für sensible Wirkstoffe wie z. B. Peptide und Proteine eignet (siehe Grafik 5), sondern daß diese zusätzlich biokompatibel, sicher und unbedenklich ist.

Die erfindungsgemäße Polymerzusammensetzung wird als Feststoff (beispielsweise in Teilchen- oder Schichtform) oder Lösung hergestellt. Je nach Herstellungsbedingungen besitzt die Polymerzusammensetzung ein Netzwerk aus korrespondierenden Anteilen organischer und anorganischer Komponenten, mit einem genau definierten Vernetzungsgrad. Mithilfe des Vernetzungsgrades wird eine bestimmte Maschenweite oder Porengröße des Netzwerks eingestellt, so daß z.B. Wirkstoffe unterschiedlichen Molekulargewichts unterschiedliche Freigabeprofile zeigen.

Bevorzugte Anwendungen der Erfindung liegt in der Herstellung von Trägersystemen für pharmazeutische Wirkstoffe, deren Freigabe kontrolliert werden soll:

Konkrete Beispiele sind:
die Einlagerung von Oligonucleotiden, Peptiden, Proteinen (z.B. Antisense-Nukleotiden, Hormonen, Releasingfaktoren, Insulin, DNA oder Vakzine) in parenterale Depotformen wie Mikropartikel oder Mini-Implantate
Mikrokapselformen bspw. von lebenden Zellen, Enzymen, Antigenen, Antikörpern oder von konventionellen biologischen Agenzien
zielgerichtete Transportformen von Cytostatika (z. B. 5-FU, Cisplatin etc.) zur Tumortherapie mittels oberflächenmodifizierten, wirkstoffbeladenen parenteralen Trägersystemen, wie Mikro-/Nanopartikeln
colonspezifische Arzneistoffformulierungen für die Resorption von Peptiden und Proteinen und für die Behandlung dort lokalisierter Erkrankungen
nanopartikuläre Carriersysteme z.B. für Psychopharmaka (z. B. Dopamin) oder Analgetika (z. B. Morphin-Analoga) an zur Überwindung der Blut-Hirn-Schranke;
Einbringung bspw. von endothelialen Wachstumsfaktoren oder Antibiotika in polymere Wundauflagen zur Geweberegeneration;
Anwendung als transdermale Darreichungsform

Im nachfolgenden sei die Erfindung an Beispielen näher erläutert, ohne sie jedoch darauf zu beschränken:

### Ausführungsbeispiele

### Beispiel 1:

### Herstellung eines Vergleichsbeispiels auf Basis von Gelatine mit inkorporiertem Wirkstoff

In einem geeigneten Doppelmantelgefäß mit Kühl- und Heizvorrichtung, Rührer, Temperaturmessung, pH-Wert-Meßgerät werden 20 g Gelatine in 180 ml destilliertem Wasser eingequollen. Die Quellzeit beträgt ca. 2 Stunden bei einer Temperatur ≤ 15 °C. Anschließend wird die gequollene Gelatine innerhalb von 30 Minuten unter Rühren bei ca. 200 U / min bei ca. 45 °C geschmolzen.
Nach dem Schmelzen wird die Rührerdrehzahl auf 400 U / min erhöht und unter weiterem Rühren 0,5 g des Modellfarbstoffes S 0120 eingetragen.
Die Temperatur wird auf 60 °C erhöht und unter Beibehaltung der Rührerdrehzahl 240 Minuten weitergerührt.
Zum Abschluß wird die Lösung in einem Sprühtrockner B-191 der Firma Büchi AG in bekannter Weise getrocknet. Die erhaltenen Mikropartikel werden im Vakuum nachgetrocknet und in geeigneten Gefäßen unter Feuchtigkeitsausschluß aufbewahrt. Die Ausbeute an Mikropartikeln liegt bei ca. 47 %.
Der Beladungsgrad mit Farbstoff liegt bei 2,2 Gewichtsprozent (Gew.-%).
Die Mikropartikel sind ca. 4, 5 µm groß.

### Beispiel 2:

### Herstellung eines Vergleichsbeispiels auf Basis von Albumin mit inkorporiertem Wirkstoff

In einem geeigneten Doppelmantelgefäß mit Kühl- und Heizvorrichtung, Rührer, Temperaturmessung, pH-Wert-Meßgerät werden 10 g Albumin (BSA) in 190 ml destilliertem Wasser eingequollen. Die Quellzeit beträgt ca. 2 Stunden bei einer Temperatur ≤ 15 °C. Anschließend wird das gequollene Albumin innerhalb von 10 Minuten unter Rühren bei ca. 200 U / min bei ca. 35 °C geschmolzen.
Nach dem Schmelzen wird die Rührerdrehzahl auf 400 U / min erhöht und unter weiterem Rühren 0,25 g des Modellfarbstoffes S 0120 eingetragen.
Die Temperatur wird auf 40 °C erhöht und unter Beibehaltung der Rührerdrehzahl 60 Minuten weitergerührt.
Zum Abschluß wird die Lösung in einem Sprühtrockner B-191 der Firma Büchi AG in bekannter Weise getrocknet.
Die erhaltenen Mikropartikel werden im Vakuum nachgetrocknet und in geeigneten Gefäßen unter Feuchtigkeitsausschluß aufbewahrt.
Die Ausbeute an Mikropartikeln liegt bei ca. 50 %.
Der Beladungsgrad mit Farbstoff liegt bei 2,1 Gew.-%.
Die Mikropartikel sind ca. 4, 5 µm groß.

Herstellung einer erfindungsgemäßen Polymerzusammensetzung auf Basis von Gelatine mit inkorporiertem Modellwirkstoff

Als Modellwirkstoff wird der Farbstoff S 0120 der Firma FEW Chemicals verwendet
(2-┌7-┌1,3-Dihydro-3,3-dimethyl-5-sulfo-1-(4-sulfobutyl)-2H-indol-2-ylidene┘-hepta-1,3,5-trienyl┘-3,3-dimethyl-5-sulfo-1-(4-sulfobutyl)-3H-indolium hydroxide, innersalt, tripotassium salt
Summenformel : **C**_{**35**}**H**_{**41**}**K**_{**31**}**N**_{**2**}**O**_{**12**}**S**_{**4**}
Molmasse : 927,25 g mol⁻¹

### Beispiel 3:

In einem geeigneten Doppelmantelgefäß mit Kühl- und Heizvorrichtung, Rührer, Temperaturmessung, pH-Wert-Meßgerät werden 20 g Gelatine in 180 ml destilliertem Wasser eingequollen. Die Quellzeit beträgt ca. 2 Stunden bei einer Temperatur ≤ 15 °C. Anschließend wird die gequollene Gelatine innerhalb von 30 Minuten unter Rühren bei ca. 200 U / min bei ca. 45 °C geschmolzen.
Nach dem Schmelzen wird die Rührerdrehzahl auf 400 U / min erhöht und unter weiterem Rühren 0,5 g des Modellfarbstoffes S 0120 eingetragen.
Anschließend werden 18,5 g eines 10 Gew.-%igen Mischsoles 1 (hergestellt aus 25 Teilen Tetraethoxysilan / 75 Teilen Glycidoxypropyltriethoxysilan, berechnet für eine vollständige Kondensation) hinzugefügt. Die Temperatur wird auf 60 °C erhöht und unter Beibehaltung der Rührerdrehzahl 240 Minuten weitergerührt.
Zum Abschluß wird die Lösung in einem Sprühtrockner B-191 der Firma Büchi AG in bekannter Weise getrocknet.
Die erhaltenen Mikropartikel werden im Vakuum nachgetrocknet und in geeigneten Gefäßen unter Feuchtigkeitsausschluß aufbewahrt.
Die Ausbeute an Mikropartikeln liegt bei ca.45 %.
Der Beladungsgrad mit Farbstoff liegt bei 2,4 Gew.-%.
Die Mikropartikel sind ca. 4,0 µm groß.
Der Anteil von SiO₂ in der Polymerzusammensetzung liegt bei ca. 5 Gew.-%.

### Beispiel 4:

In einem geeigneten Doppelmantelgefäß mit Kühl- und Heizvorrichtung, Rührer, Temperaturmessung, pH-Wert-Meßgerät werden 20 g Gelatine in 180 ml destilliertem Wasser eingequollen. Die Quellzeit beträgt ca. 2 Stunden bei einer Temperatur ≤ 15 °C. Anschließend wird die gequollene Gelatine innerhalb von 30 Minuten unter Rühren bei ca. 200 U / min bei ca. 45 °C geschmolzen.
Nach dem Schmelzen wird die Rührerdrehzahl auf 400 U / min erhöht und unter weiterem Rühren 0,5 g des Modellfarbstoffes S 0120 eingetragen.
Anschließend werden 74 g eines 10 Gew.-%igen Mischsoles 2 (hergestellt aus 50 Teilen Tetraethoxysilan / 50 Teilen Glycidoxypropyltriethoxysilan, berechnet für eine vollständige Kondensation) hinzugefügt.
Die Temperatur wird auf 60 °C erhöht und unter Beibehaltung der Rührerdrehzahl 240 Minuten weitergerührt.
Die Lösung wird in einem Sprühtrockner B-191 der Firma Büchi AG in bekannter Weise getrocknet. Die erhaltenen Mikropartikel werden im Vakuum nachgetrocknet und in geeigneten Gefäßen unter Feuchtigkeitsausschluß aufbewahrt. Die Ausbeute an Mikropartikeln liegt bei ca. 65 %.
Der Beladungsgrad mit Farbstoff liegt bei 2,4 Gew.-%.
Die Mikropartikel sind ca. 4,0 µm groß.
Der Anteil von SiO₂ in der Polymerzusammensetzung liegt bei ca. 15 Gew.-%.

Herstellung einer erfindungsgemäßen Polymerzusammensetzung auf Basis von Albumin mit inkorporiertem Modellwirkstoff

### Beispiel 5:

In einem geeigneten Doppelmantelgefäß mit Kühl- und Heizvorrichtung, Rührer, Temperaturmessung, pH-Wert-Meßgerät werden 10 g Albumin (BSA) in 190 ml destilliertem Wasser eingequollen. Die Quellzeit beträgt ca. 2 Stunden bei einer Temperatur ≤ 15 °C. Anschließend wird das gequollene Albumin innerhalb von 10 Minuten unter Rühren bei ca. 200 U / min bei ca. 35 °C geschmolzen.
Nach dem Schmelzen wird die Rührerdrehzahl auf 400 U / min erhöht und unter weiterem Rühren 0,25 g des Modellfarbstoffes S 0120 eingetragen.

Anschließend werden 3,8 g eines 10 Gew.-%igen Mischsoles 1 (hergestellt aus 25 Teilen Tetraethoxysilan / 75 Teilen Glycidoxypropyltriethoxysilan, berechnet für eine vollständige Kondensation) hinzugefügt.
Die Temperatur wird auf 40 °C erhöht und unter Beibehaltung der Rührerdrehzahl 60 Minuten weitergerührt.
Zum Abschluß wird die Lösung in einem Sprühtrockner B-191 der Firma Büchi AG in bekannter Weise getrocknet. Die erhaltenen Mikropartikel werden im Vakuum nachgetrocknet und in geeigneten Gefäßen unter Feuchtigkeitsausschluß aufbewahrt.
Die Ausbeute an Mikropartikeln liegt bei ca.55 %.
Der Beladungsgrad mit Farbstoff liegt bei 2,35 Gew.-%.
Die Mikropartikel sind ca. 4,0 µm groß.
Der Anteil von SiO₂ in der Polymerzusammensetzung liegt bei ca. 4,5 Gew.-%.

### Beispiel 6:

In einem geeigneten Doppelmantelgefäß mit Kühl- und Heizvorrichtung, Rührer, Temperaturmessung, pH-Wert-Meßgerät werden 10 g Albumin (BSA) in 190 ml destilliertem Wasser eingequollen. Die Quellzeit beträgt ca. 2 Stunden bei einer Temperatur ≤ 15 °C. Anschließend wird das gequollene Albumin innerhalb von 10 Minuten unter Rühren bei ca. 200 U / min bei ca. 35 °C geschmolzen.
Nach dem Schmelzen wird die Rührerdrehzahl auf 400 U / min erhöht und unter weiterem Rühren 0,25 g des Modellfarbstoffes S 0120 eingetragen.
Anschließend werden 15,2 g eines 10 Gew.-%igen Mischsoles 3 (hergestellt aus 50 Teilen Tetraethoxysilan / 50 Teile Glycidoxypropyltriethoxysilan, berechnet für eine vollständige Kondensation) hinzugefügt.
Die Temperatur wird auf 40 °C erhöht und unter Beibehaltung der Rührerdrehzahl 60 Minuten weitergerührt.
Zum Abschluß wird die Lösung in einem Sprühtrockner B-191 der Firma Büchi AG in bekannter Weise getrocknet. Die erhaltenen Mikropartikel werden im Vakuum nachgetrocknet und in geeigneten Gefäßen unter Feuchtigkeitsausschluß aufbewahrt.
Die Ausbeute an Mikropartikeln liegt bei ca. 62 %.
Der Beladungsgrad mit Farbstoff liegt bei 2,3 Gew.-%.
Die Mikropartikel sind ca. 4,0 µm groß.
Der Anteil von SiO₂ in der Polymerzusammensetzung liegt bei ca . 10, 5 Gew.-%.

Herstellung einer erfindungsgemäßen Polymerzusammensetzung auf Basis von Albumin mit inkorporiertem Modellwirkstoff Als weiterer Modellwirkstoff wird fluoreszenzmarkiertes Dextran der Sigma-Aldrich Chemie GmbH verwendet.

### Beispiel 7:

In einem geeigneten Doppelmantelgefäß mit Kühl- und Heizvorrichtung, Rührer, Temperaturmessung, pH-Wert-Meßgerät werden 10 g Albumin (BSA) in 190 ml destilliertem Wasser eingequollen. Die Quellzeit beträgt ca. 2 Stunden bei einer Temperatur ≤ 15 °C. Anschließend wird das gequollene Albumin innerhalb von 10 Minuten unter Rühren bei ca. 200 U / min bei ca. 35 °C geschmolzen.
Nach dem Schmelzen wird die Rührerdrehzahl auf 400 U / min erhöht und unter weiterem Rühren 0,25 g FITC-Dextran mit der Molmasse von 150000 g/ Mol eingetragen.
Anschließend werden 38 g eines 10 Gew.-%igen Mischsoles 2 (hergestellt aus 50 Teile Tetraethoxysilan / 50 Teile Glycidoxypropyltriethoxysilan, berechnet für eine vollständige Kondensation) hinzugefügt.
Die Temperatur wird auf 40 °C erhöht und unter Beibehaltung der Rührerdrehzahl 60 Minuten weitergerührt.

Zum Abschluß wird die Lösung in einem Sprühtrockner B-191 der Firma Büchi AG in bekannter Weise getrocknet.
Danach werden die erhaltenen Mikropartikel im Vakuum nachgetrocknet und in geeigneten Gefäßen unter Feuchtigkeitsausschluß aufbewahrt.
Die Ausbeute an Mikropartikeln liegt bei ca. 70 %.
Der Beladungsgrad mit FITC-Dextran 150000 liegt bei 2,15 Gew.-%.
Die Mikropartikel sind ca. 4,0 µm groß.
Der Anteil von SiO₂ in der Polymerzusammensetzung liegt bei ca. 15 Gew.-%.

### Beispiel 8:

In einem geeigneten Doppelmantelgefäß mit Kühl- und Heizvorrichtung, Rührer, Temperaturmessung, pH-Wert-Meßgerät werden 20 g Gelatine in 180 ml destilliertem Wasser eingequollen. Die Quellzeit beträgt ca. 2 Stunden bei einer Temperatur ≤ 15 °C. Anschließend wird die gequollene Gelatine innerhalb von 30 Minuten unter Rühren bei ca. 200 U / min bei ca. 45 °C geschmolzen.
Nach dem Schmelzen wird die Rührerdrehzahl auf 400 U / min erhöht und unter weiterem Rühren 0,5 g FITC-Dextran mit der Molmasse von 70000 g / Mol eingetragen.
Anschließend werden 18,5 g eines 10 Gew.-%igen Mischsoles 2 (hergestellt aus 25 Teile Tetraethoxysilan / 75 Teile Glycidoxypropyltriethoxysilan, berechnet für eine vollständige Kondensation) hinzugefügt.
Die Temperatur wird auf 60 °C erhöht und unter Beibehaltung der Rührerdrehzahl 240 Minuten weitergerührt.
Zum Abschluß wird die Lösung am Sprühtrockner in bekannter Weise getrocknet.
Danach werden die erhaltenen Mikropartikel im Vakuum nachgetrocknet und in geeigneten Gefäßen unter Feuchtigkeitsausschluß aufbewahrt.
Die Ausbeute an Mikropartikeln liegt bei ca. 48 %.
Der Beladungsgrad mit FITC-Dextran 70000 liegt bei 2,25 Gew.-%.
Die Mikropartikel sind ca. 3,5 µm groß.
Der Anteil von SiO₂ in der Polymerzusammensetzung liegt bei ca. 5 Gew.-%.

### Beispiel 9 :

In einer Freigabeapparatur Thermomixer comfort der Firma Eppendorf wird das in vitro-Freigabeverhalten der Mikropartikel der Beispiele 1 - 6 untersucht.
In 2 ml Eppendorf-Reaktionsgefäße werden jeweils 4 mg Mikropartikel ( MP ) und 2 ml dest. Wasser eingefüllt. Das in vitro-Freigabeverhalten wird bei einer Temperatur von 37 °C und einer Mischfrequenz von 500 U / min und einem Intervallmix 60 Sekunden Unterbrechung / 60 Sekunden Mischphase über 3 Wochen verfolgt.

Die Ergebnisse sind den Tabellen 1 und 2 zu entnehmen und den Grafiken 1 und 2 zu entnehmen.

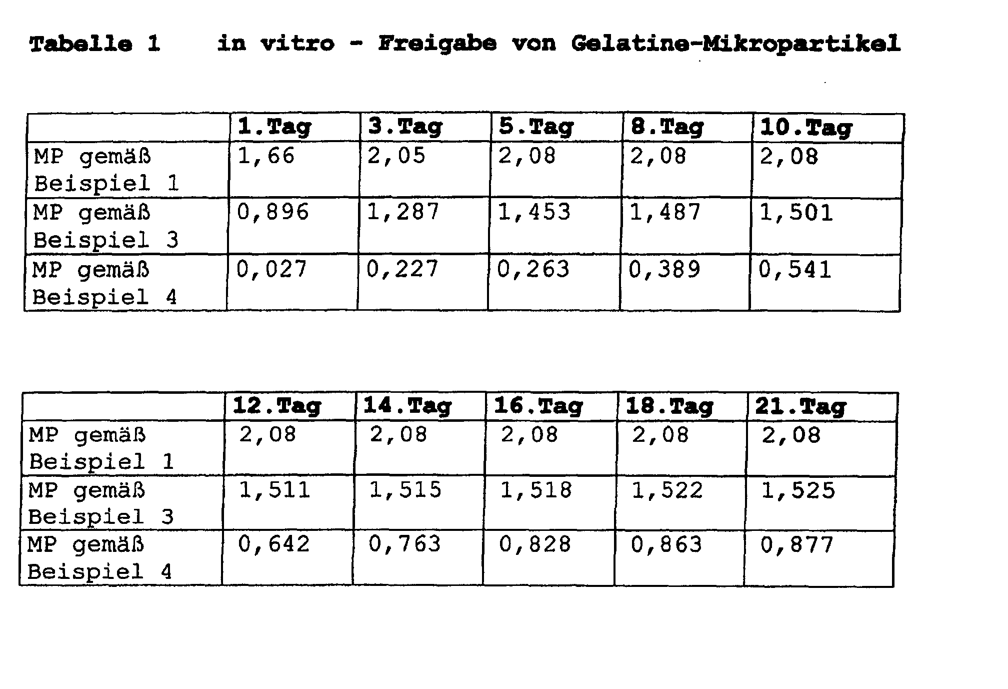

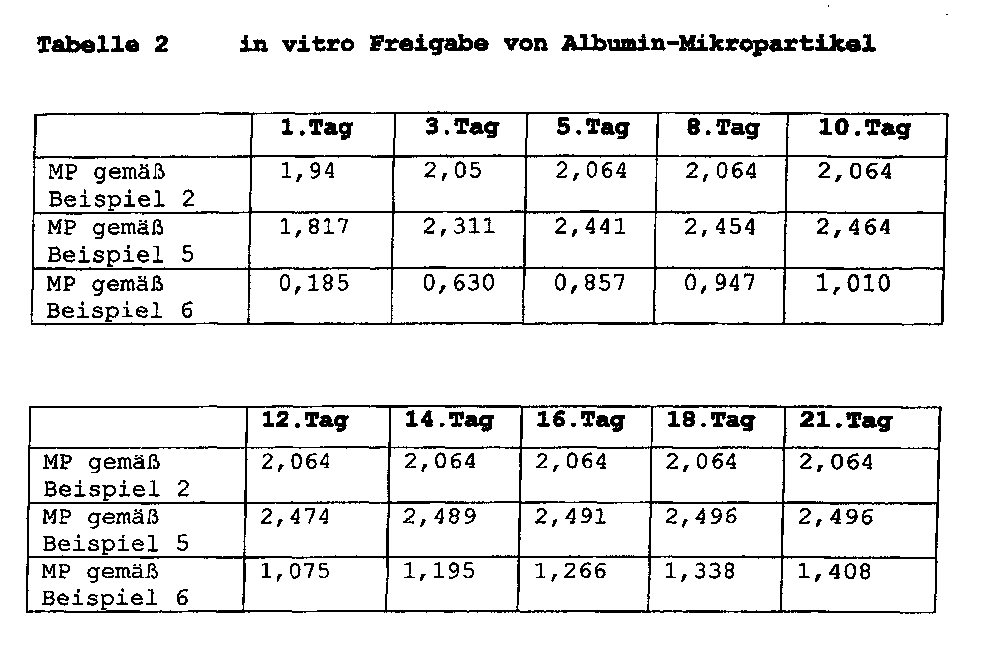

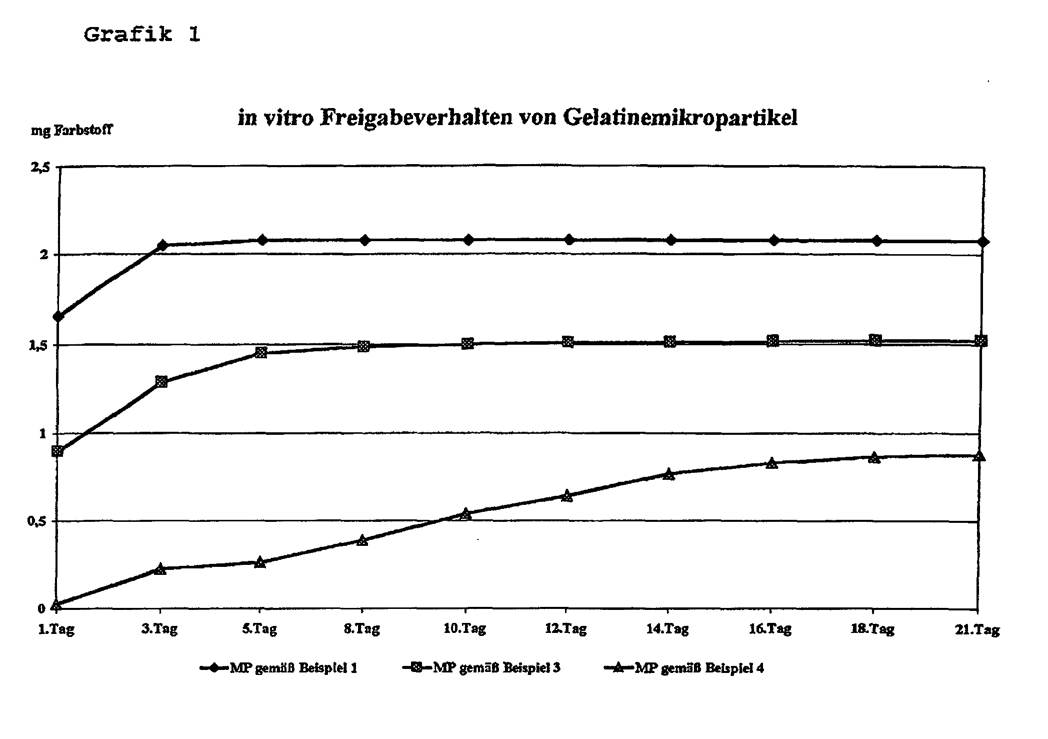

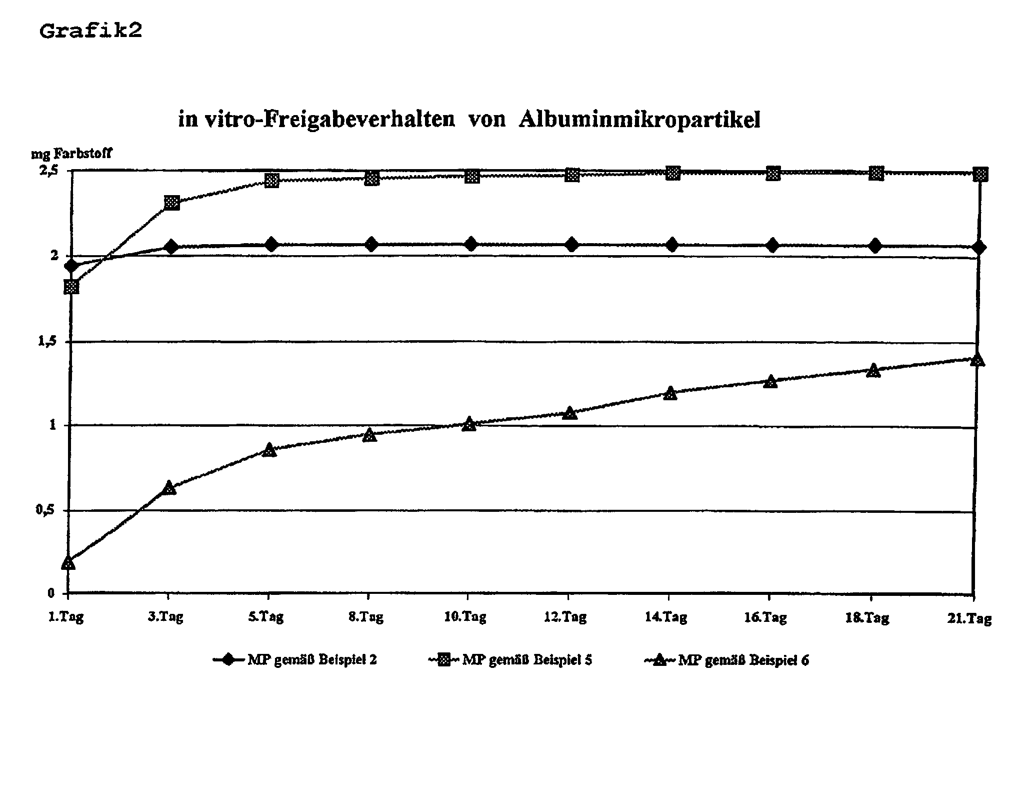

### Beispiel 10

### Stabilitätsuntersuchung

In einer Freigabeapparatur Thermomixer comfort der Firma Eppendorf wird das in vitro-Freigabeverhalten der 4 Wochen gelagerten Mikropartikel der Beispiele 1 - 6 untersucht.
In 2 ml Eppendorf-Reaktionsgefäße werden jeweils 4 mg Mikropartikel ( MP) und 2 ml dest. Wasser eingefüllt.
Das in vitro-Freigabeverhalten wird bei einer Temperatur von 37 °C und einer Mischfrequenz von 500 U / min und einem Intervallmix 60 Sekunden Unterbrechung / 60 Sekunden Mischphase über 4 Wochen verfolgt.
Die Ergebnisse sind den Tabellen 3 und 4 sowie den Grafiken 3 und 4 zu entnehmen.

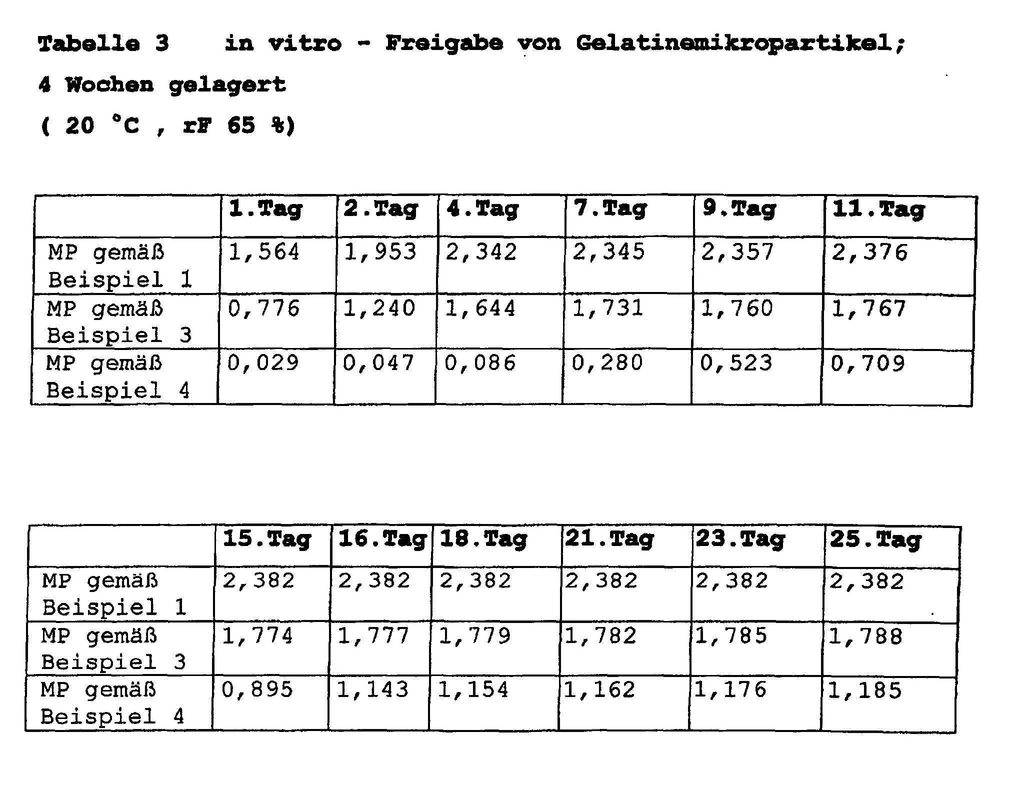

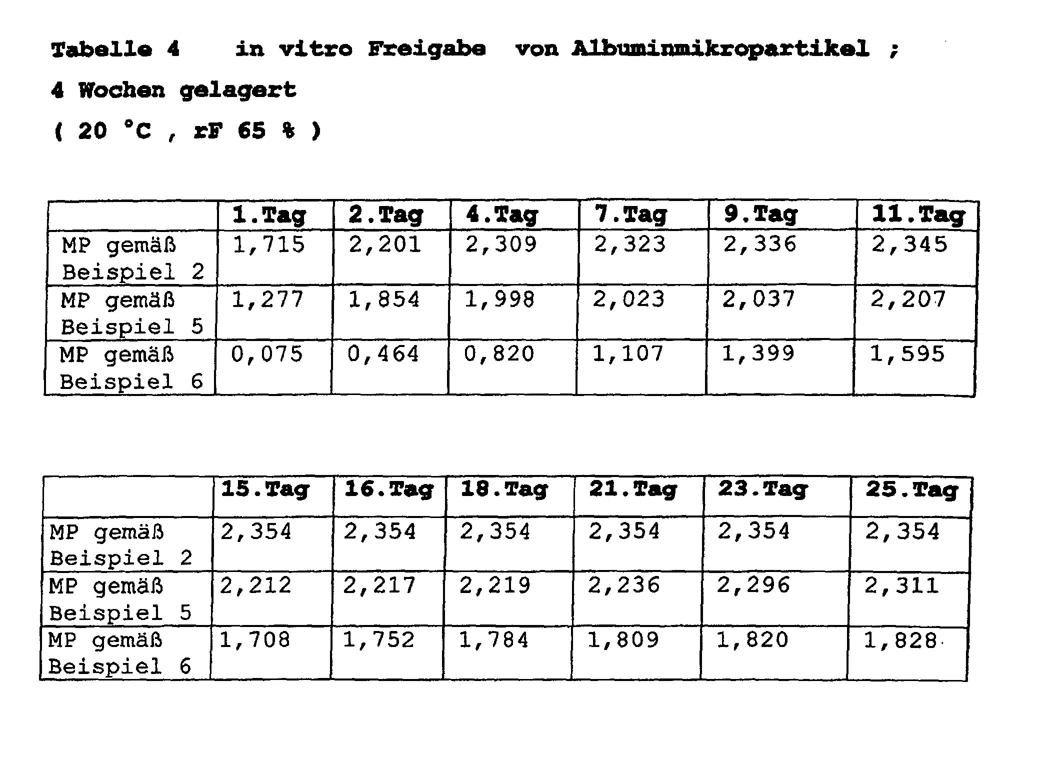

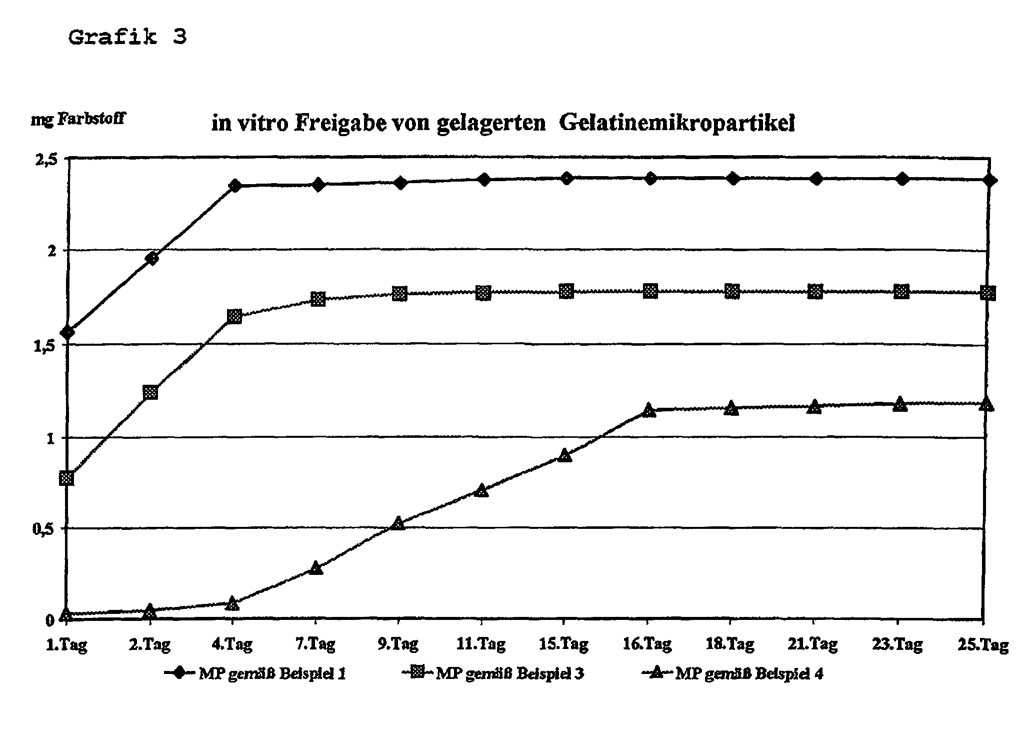

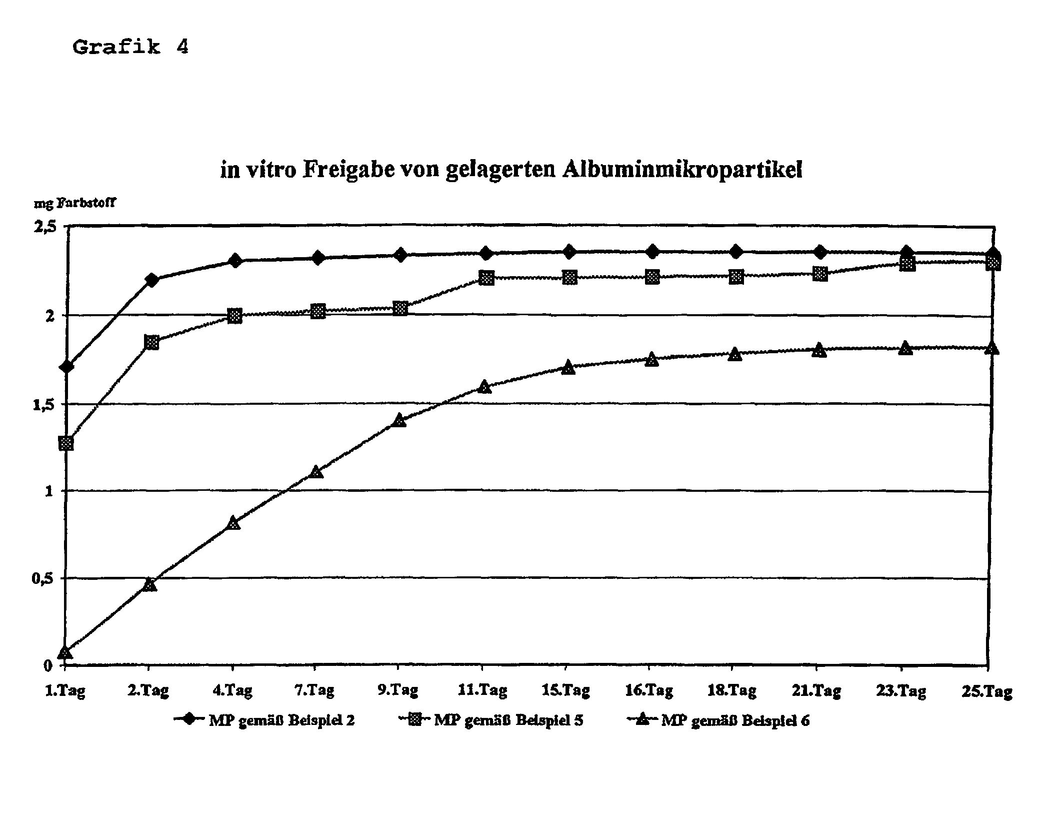

Herstellung einer erfindungsgemäßen Polymerzusammensetzung auf Basis von Gelatine mit inkorporiertem Modellwirkstoff Als Wirkstoff wird Bovines Serum Albumin (BSA) der Sigma-Aldrich Chemie GmbH verwendet.

### Beispiel 11 :

In einem geeigneten Doppelmantelgefäß mit Kühl- und Heizvorrichtung, Rührer, Temperaturmessung, pH-Wert-Meßgerät werden 20 g Gelatine in 180 ml destilliertem Wasser eingequollen. Die Quellzeit beträgt ca. 2 Stunden bei einer Temperatur ≤ 15 °C. Anschließend wird das gequollene Albumin innerhalb von 10 Minuten unter Rühren bei ca. 200 U / min bei ca. 35 °C geschmolzen.
Nach dem Schmelzen wird die Rührerdrehzahl auf 400 U / min erhöht und unter weiterem Rühren 0,5 g Albumin (BSA) eingetragen.
Anschließend werden 18,5g eines 10 Gew.-%igen Mischsoles 2 (hergestellt aus 50 Teile Tetraethoxysilan / 50 Teile Glycidoxypropyltriethoxysilan, berechnet für eine vollständige Kondensation) hinzugefügt.
Die Temperatur wird auf 40 ° C erhöht und unter Beibehaltung der Rührerdrehzahl 60 Minuten weitergerührt. Zum Abschluß wird die Lösung in einem Sprühtrockner B-191 der Firma Büchi AG in bekannter Weise getrocknet.
Danach werden die erhaltenen Mikropartikel im Vakuum nachgetrocknet und in geeigneten Gefäßen unter Feuchtigkeitsausschluß aufbewahrt.
Die Ausbeute an Mikropartikeln liegt bei ca. 70 %.
Der Beladungsgrad mit BSA liegt bei 2,15 Gew.-%.
Die Mikropartikel sind ca. 4,0 µm groß.
Der Anteil von SiO₂ in der Polymerzusammensetzung liegt bei ca. 8 Gew.-%.

### Beispiel 12 :

In einem geeigneten Doppelmantelgefäß mit Kühl- und Heizvorrichtung, Rührer, Temperaturmessung, pH-Wert-Meßgerät werden 20 g Gelatine in 180 ml destilliertem Wasser eingequollen. Die Quellzeit beträgt ca. 2 Stunden bei einer Temperatur ≤ 15 °C. Anschließend wird das gequollene Albumin innerhalb von 10 Minuten unter Rühren bei ca. 200 U / min bei ca. 35 °C geschmolzen.
Nach dem Schmelzen wird die Rührerdrehzahl auf 400 U / min erhöht und unter weiterem Rühren 0,5 g Albumin (BSA) eingetragen.
Anschließend werden 74 g eines 10 Gew.-%igen Mischsoles 2 (hergestellt aus 50 Teile Tetraethoxysilan / 50 Teile Glycidoxypropyltriethoxysilan, berechnet für eine vollständige Kondensation) hinzugefügt.
Die Temperatur wird auf 40 ° C erhöht und unter Beibehaltung der Rührerdrehzahl 60 Minuten weitergerührt. Zum Abschluß wird die Lösung in einem Sprühtrockner B-191 der Firma Büchi AG in bekannter Weise getrocknet.
Danach werden die erhaltenen Mikropartikel im Vakuum nachgetrocknet und in geeigneten Gefäßen unter Feuchtigkeitsausschluß aufbewahrt.
Die Ausbeute an Mikropartikeln liegt bei ca. 70 %.
Der Beladungsgrad mit BSA liegt bei 2,15 Gew.-%.
Die Mikropartikel sind ca. 4,0 µm groß.
Der Anteil von SiO₂ in der Polymerzusammensetzung liegt bei ca. 15 Gew.-%.

### Beispiel 13:

In einer Freigabeapparatur Thermomixer comfort der Firma Eppendorf wird das in vitro-Freigabeverhalten der Mikropartikel der Beispiele 11 - 12 im Vergleich mit unvernetzten Mikropartikeln untersucht.
In 2 ml Eppendorf-Reaktionsgefäße werden jeweils 4 mg Mikropartikel ( MP ) und 2 ml Pufferlösung pH 7,4 eingefüllt.
Das in vitro-Freigabeverhalten wird bei einer Temperatur von 37 °C und einer Mischfrequenz von 500 U / min und einem Intervallmix 60 Sekunden Unterbrechung / 60 Sekunden Mischphase über 3 Wochen verfolgt.
Die Ergebnisse ist der Tabelle 5 zu entnehmen. siehe auch Grafik 5

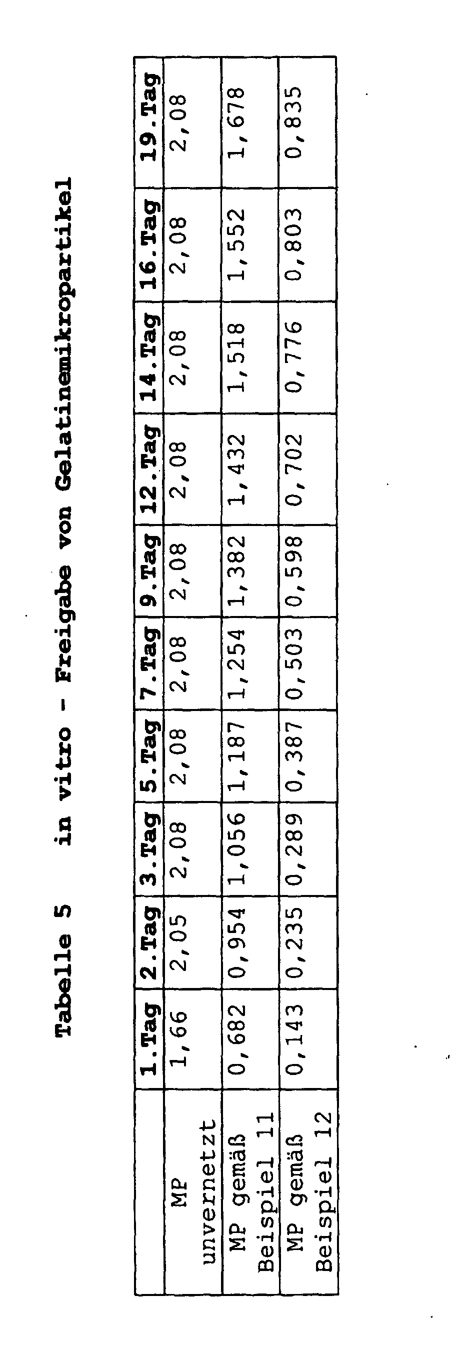

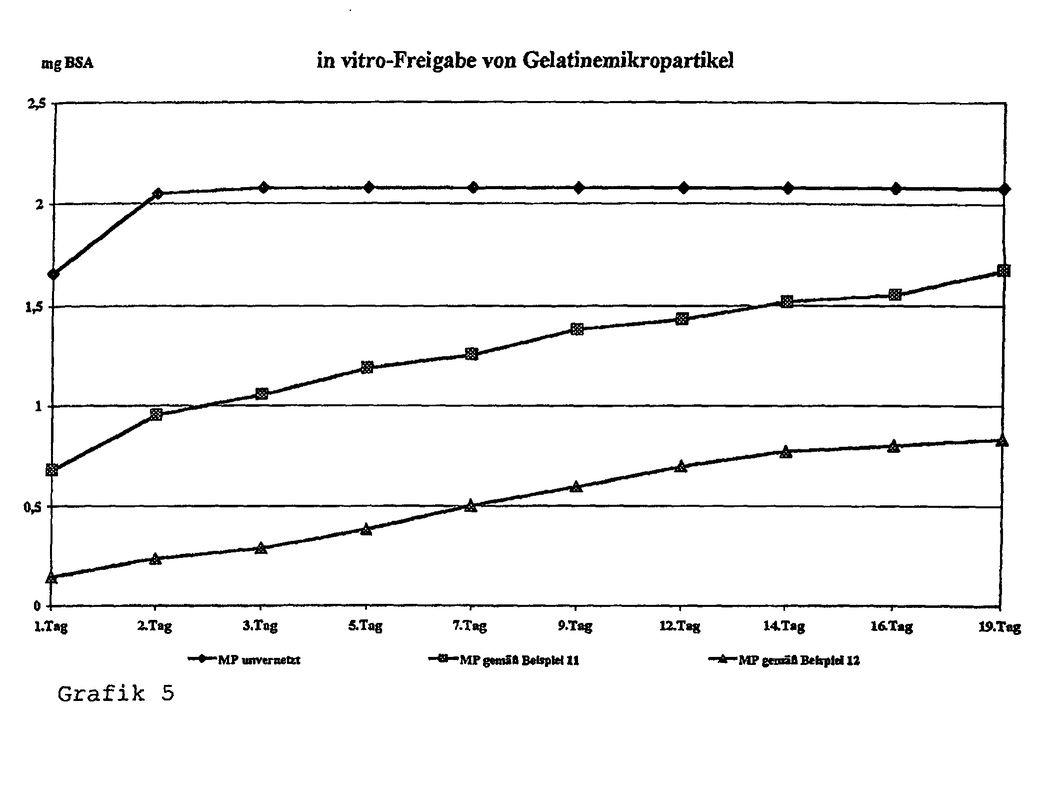

Herstellung einer Polymerzusammensetzung mit Gelatine als Ausgangsstoff
für die klassische Mikroverkapselung

### Beispiel 14 :

In einem geeigneten Doppelmantelgefäß mit Kühl- und Heizvorrichtung, Rührer, Temperaturmessung werden 10 g Gelatine in 100 ml destilliertem Wasser eingequollen.
Die Quellzeit beträgt ca. 2 Stunden bei 15 °C .
Anschließend wird die gequollene Gelatine innerhalb von 50 Minuten unter Rühren ca. 200 U / min bei ca. 45 °C geschmolzen.
Nach dem Schmelzen wird die Rührgeschwindigkeit auf ca. 400 U / min erhöht und unter Beibehaltung der Temperatur von 45 °C werden 2,6 g eines 10 Gew.-%igen Mischsoles 1 (hergestellt aus 25 Teile Tetraethoxysilan / 75 Teile Glycidoxypropyltriethoxysilan ) zugesetzt.
Nach erfolgter Zugabe wird 60 Minuten weitergerührt. Anschließend wird die erhaltene Polymerzusammensetzung in einem Sprühtrockner getrocknet.
Die Polymerzusammensetzung hat einen SiOₓ-Anteil von 2,5 Gew.-%.
Das erhaltene Pulver wird in geeigneten Gefäßen unter Feuchtigkeitsausschluß aufbewahrt.
Die nach diesem Verfahren hergestellte Polymerzusammensetzung kann z.B. als Wandmaterial in Kombination mit einer weiteren Komponente in bekannter Weise ( "Microcapsule Processing and Technology" (A. Kondo ed. J. Wade Van Valkenburg, M. Dekker, Inc. N.Y 1979)) für die klassische Mikroverkapselung eingesetzt werden.

## Patentansprüche

1. Polymerzusammensetzung, die wasserlösliche Polymere enthält, **dadurch gekennzeichnet, dass** die wasserlöslichen Polymere über die Hauptkette und/oder die Seitenketten mit kovalent gebundenen Siliziumoxid-Bausteinen vernetzt sind.

2. Polymerzusammensetzung gemäß Anspruch 1, die als Trägermaterial für biologisch aktive Agenzien dient.

3. Polymerzusammensetzung gemäß Anspruch 1 oder 2, bei der die wasserlöslichen Polymere biokompatible und/oder bioabbaubare Polymere sind und zu den Polysacchariden, Proteinen oder Zellulosederivaten gehören.

4. Verfahren zur Herstellung einer Polymerzusammensetzung gemäß einem der Ansprüche 1 bis 3, bei dem eine wässrige Polymerlösung mit funktionellen Alkoxysilanen, deren Hydrolysaten und den aus ihnen gebildeten Kondensationsprodukten oder den aus ihnen gebildeten funktionellen Siliziumoxidsolen versetzt wird.

5. Verfahren gemäß Anspruch 4, bei dem die Alkoxysilane die allgemeine Formel Si(OR)ₓR¹_{Y} besitzen, wobei R ein oder verschiedene C₁-C₄-Alkylreste, R¹ ein oder verschiedene Alkyl-, Aryl- oder Alkylarylreste, optional mit einer funktionellen Gruppe, x eine Zahl im Bereich 1 bis 4 und y eine Zahl im Bereich 0 bis 3 umfassen, wobei ein Mindestgehalt an funktionellem Silan von 0,1 Mol vorliegt.

6. Verfahren gemäß Anspruch 4 und 5, bei dem der wässrigen Polymerlösung ein Mischsol aus funktionellen und nicht-funktionellen Siliziumoxidsolen zugesetzt wird.

7. Verfahren gemäß einem der Ansprüche 4 bis 6, bei dem die Polymerlösung Polysaccharide, Proteine oder Zellulosederivate enthält.

8. Verfahren gemäß einem der Ansprüche 4 bis 7, bei dem der Polymerlösung biologisch aktive Agenzien, bevorzugt pharmazeutische Wirkstoffe zugesetzt werden.

9. Verfahren zur Einlagerung biologisch aktiver Agenzien, bei dem eine Polymerzusammensetzung aus wasserlöslichen Polymeren und kovalent gebundenen Siliziumoxid-Bausteinen durch Reaktion von in der Hauptkette und/oder in den Seitenketten der wasserlöslichen Polymere vorhandenen und/oder durch zusätzliche chemische Modifizierung der wasserlöslichen Polymere erzeugten funktionellen Gruppen mit funktionellen Metallalkoxiden oder deren Hydrolysaten gebildet wird, wobei die biologisch aktiven Agenzien den wasserlöslichen Polymeren in flüssiger Phase oder der Polymerzusammensetzung in fester Phase zugesetzt werden.

10. Verfahren gemäß Anspruch 9, bei dem zur Herstellung der Polymerzusammensetzung eine wässrige Polymerlösung aus Polysacchariden, Proteinen oder Zellulosederivaten, deren Polymere in der Hauptkette oder in den Seitenketten funktionelle Gruppen enthalten, mit durch funktionellen Silanen der allgemeinen Formel Si(OR)ₓR¹_{y}, oberflächenmodifizierten SiOₓ-Solen oder SiOₓ-Dispersionen versetzt wird, wobei R einen C₁ - C₄-Alkylrest bedeutet, R¹ einen Alkyl-, Aryl- oder Alkylarylrest, optional mit einer reaktionsfähigen Gruppe, bedeutet, R und R¹ gleich oder verschieden sind, und x für eine Zahl von 2 bis 3 und y für eine Zahl von 1 bis 2 stehen.

11. Verwendung einer Polymerzusammensetzung gemäß einem der Ansprüche 1 bis 3 als Trägermaterial, z. B. als Matrix, welche als Partikel, Wirkstoffdepot oder Schicht vorliegt und der kontrollierten Freigabe der biologisch aktiven Agenzien dient.

12. Verwendung einer Polymerzusammensetzung gemäß einem der Ansprüche 1 bis 3 als Wandmaterial zum Verkapseln biologisch aktiver Agenzien oder biologischen Materials zur kontrollierten Freigabe oder zum Schutz desselben.

## Claims

1. A polymer composition which contains water-soluble polymers, **characterized in that** the water-soluble polymers are cross-linked via the main chain and/or the side chains with covalently bonded silicon oxide units.

2. A polymer composition according to claim 1, which serves as a carrier material for biologically active agents

3. A polymer composition according to claim 1 or 2, in which the water-soluble polymers are bio-compatible and/or bio-degradable polymers and pertain to the polysaccharides, proteins or cellulose derivatives.

4. A process for making a polymer composition according to any of claims 1 to 3, in which an aqueous polymer solution is mixed with functional alkoxysilanes, their hydrolyslates and the condensation products formed therefrom or the functional silicon oxide sols formed therefrom.

5. A process according to claim 4, in which the alkoxysilanes have the general formula Si(OR)ₓR¹_{y}, wherein R comprises one or various C₁-C₄ alkyl residues, R¹ comprises one or various alkyl, aryl or alkylaryl residues, optionally with a functional group, x is a number in the range 1 to 4 and y is a number in the range 0 to 3, wherein a minimum content of functional silane of 0.1 mole is present.

6. A process according to claim 4 or 5, in which the aqueous polymer solution is composed of a mixed sol of functional and non-functional silicon oxide sols.

7. A process according to any of claims 4 to 6, in which the polymer solution contains polysaccharides, proteins or cellulose derivatives.

8. A process according to any of claims 4 to 7, in which biologically active agents, especially pharmaceutical materials, are added to the polymer solution.

9. A method of incorporating biologically active agents, in which a polymer composition is formed from water-soluble polymers and covalently bonded silicon oxide units by reaction of functional groups present in the main chain and/or in the side chains of the water-soluble polymers and/or created by additional chemical modification of the water-soluble polymers with functional metal alkoxides or their hydrolysates, wherein the biologically active agents are added to the water-soluble polymers in the liquid phase or to the polymer composition in the solid phase.

10. A method according to claim 9, in which, in order to produce the polymer composition, an aqueous polymer solution of poly saccharides, proteins or cellulose derivative, whose polymers have functional groups in the main chain or in the side chains, has added thereto functional silanes of the general formula Si(OR)ₓR¹_{y}, surface modified SiOₓ sols or SiOₓ dispersions, wherein R signifies a C₁ - C₄ alkyl residue, R¹ an alkyl, aryl or alkylaryl residue, optionally with a group capable of reaction, R and R¹ are the same or different, and x stands for a number from 2 to 3 and y for a number from 1 to 2.

11. Use of a polymer composition according to any of claims 1 to 3 as a carrier material, e.g. as a matrix, which is present as particles, a time-release drug or a layer and serves for controlled release of the biologically active agents.

12. Use of a polymer composition according to any of claims 1 to 3 as a wall material for encapsulating biologically active agents or biological materials for controlled release or protection thereof.

## Revendications

1. Composition polymère, qui contient des polymères solubles dans l'eau, **caractérisée en ce que** les polymères solubles dans l'eau sont réticulés par la chaîne principale et/ou les chaînes latérales avec des éléments oxyde de silicium liés de manière covalente.

2. Composition polymère selon la revendication 1, qui sert de matériau support pour des agents biologiquement actifs.

3. Composition polymère selon la revendication 1 ou 2, dans laquelle les polymères solubles dans l'eau sont des polymères biocompatibles et/ou biodégradables et appartiennent aux polysaccharides, protéines ou dérivés de la cellulose.

4. Procédé de préparation d'une composition polymère selon l'une quelconque des revendications 1 à 3, dans lequel on ajoute à une solution aqueuse de polymère, des alcoxysilanes fonctionnels, leurs hydrolysats et les produits de condensation formés à partir de ceux-ci ou les sols d'oxyde de silicium fonctionnels formés à partir de ceux-ci.

5. Procédé selon la revendication 4, dans lequel les alcoxysilanes possèdent la formule générale Si(OR)ₓR¹_{y}, où R représente un ou différents restes alkyle en C₁-C₄, R¹ représente un ou différents restes alkyle, aryle ou alkylaryle, le cas échéant avec un radical fonctionnel, x est un nombre situé dans l'intervalle allant de 1 à 4 et y un nombre situé dans l'intervalle allant de 0 à 3, où une teneur minimale en silane fonctionnel est de 0,1 mole.

6. Procédé selon les revendications 4 et 5, dans lequel on ajoute à la solution aqueuse de polymère, un sol mixte de sols d'oxyde de silicium fonctionnel et non fonctionnel.

7. Procédé selon l'une quelconque des revendications 4 à 6, dans lequel la solution de polymère contient des polysaccharides, protéines ou dérivés de la cellulose.

8. Procédé selon l'une quelconque des revendications 4 à 7, dans lequel on ajoute à la solution de polymère, les agents biologiquement actifs, de préférence des agents pharmaceutiquement actifs.

9. Procédé pour introduire des agents biologiquement actifs, dans lequel une composition polymère de polymères solubles dans l'eau et d'éléments oxyde de silicium liés de manière covalente, est formée par réaction de groupes fonctionnels présents dans la chaîne principale et/ou les chaînes latérales du polymère soluble dans l'eau et/ou produits par modification chimique supplémentaire du polymère soluble dans l'eau, avec des alcoxydes métalliques fonctionnels ou leur hydrolysats, où les agents biologiquement actifs sont ajoutés aux polymères solubles dans l'eau en phase liquide ou à la composition polymère en phase solide.

10. Procédé selon la revendication 9, dans lequel pour la préparation de la composition polymère, on ajoute à une solution aqueuse de polymère constituée de polysaccharides, protéines ou dérivés de la cellulose, dont les polymères contiennent des radicaux fonctionnels dans la chaîne principale ou dans les chaînes secondaires, des silanes fonctionnels de la formule générale Si(OR)_{X}R¹_{y}, des sols de SiOₓ modifiés en surface, ou des dispersions de SiOₓ, où R représente un reste alkyle en C₁-C₄, R¹ représente un reste alkyle, aryle ou alkylaryle, le cas échéant avec un radical fonctionnel, R et R¹ sont identiques ou différents, x est un nombre allant de 2 à 3 et y un nombre allant de 1 à 2.

11. Utilisation d'une composition polymère selon l'une quelconque des revendications 1 à 3, comme matériau support, par exemple comme matrice, qui se présente sous la forme de particules, dépôt d'agent actif ou couche et sert à la libération contrôlée d'agents biologiquement actifs.

12. Utilisation d'une composition polymère selon l'une quelconque des revendications 1 à 3, comme matériau de paroi pour l'encapsulation d'agents biologiquement actifs ou de matériaux biologiques pour la libération contrôlée ou pour les protéger.
